(19) Europäisches Patentamt
European Patent Office
Office européen des brevets

(11) **EP 2 767 917 B1**

(12) **EUROPEAN PATENT SPECIFICATION**

(45) Date of publication and mention of the grant of the patent:
**13.10.2021 Bulletin 2021/41**

(51) Int Cl.:
***G16C 20/30*** *(2019.01)* ***G16C 20/10*** *(2019.01)*

(21) Application number: **13190802.2**

(22) Date of filing: **30.10.2013**

(54) **Apparatus, method, program, and storage medium for predicting acid dissociation constant**

Vorrichtung, Verfahren, Programm und Speichermedium zur Vorhersage von konstanter Spaltung

Appareil, procédé, programme et support de stockage permettant de prédire une constante dissociation d'acide

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.02.2013 JP 2013026464**

(43) Date of publication of application:
**20.08.2014 Bulletin 2014/34**

(73) Proprietor: **FUJITSU LIMITED**
**Kawasaki-shi,**
**Kanagawa 211-8588 (JP)**

(72) Inventors:
• **Sato, Hiroyuki**
**Kawasaki-shi, Kanagawa 211-8588 (JP)**
• **Matsuura, Azuma**
**Kawasaki-shi, Kanagawa 211-8588 (JP)**

(74) Representative: **Haseltine Lake Kempner LLP**
**Cheapside House**
**138 Cheapside**
**London EC2V 6BJ (GB)**

(56) References cited:
• **GHASEMI J. ET AL: "QSPR study for estimation of acidity constants of some aromatic acids derivatives using multiple linear regression (MLR) analysis", JOURNAL OF MOLECULAR STRUCTURE (THEOCHEM), ELSEVIER SCIENCE PUBLISHERS B.V., AMSTERDAM, NL, vol. 805, no. 1-3, 1 February 2007 (2007-02-01), pages 27-32, XP005868316, ISSN: 0166-1280, DOI: 10.1016/J.THEOCHEM.2006.09.026**
• **MAYER I.: "Bond order and valence indices: A personal account", JOURNAL OF COMPUTATIONAL CHEMISTRY, vol. 28, no. 1, 15 January 2007 (2007-01-15), pages 204-221, XP055120798, ISSN: 0192-8651, DOI: 10.1002/jcc.20494**
• **SORIANO E. ET AL: "Computational determination of pKa values. A comparison of different theoretical approaches and a novel procedure", JOURNAL OF MOLECULAR STRUCTURE (THEOCHEM), ELSEVIER SCIENCE PUBLISHERS B.V., AMSTERDAM, NL, vol. 684, no. 1-3, 27 September 2004 (2004-09-27), pages 121-128, XP004645914, ISSN: 0166-1280, DOI: 10.1016/J.THEOCHEM.2004.06.041**
• **SARACINO G. A. A. ET AL: "Absolute pKa determination for carboxylic acids using density functional theory and the polarizable continuum model", CHEMICAL PHYSICS LETTERS, vol. 373, no. 3-4, 1 May 2003 (2003-05-01), pages 411-415, XP055120939, ISSN: 0009-2614, DOI: 10.1016/S0009-2614(03)00607-9**
• **None**

**Description**

FIELD

The embodiment discussed herein is related to prediction of an acid dissociation constant.

BACKGROUND

$pK_a$ is a constant that represents acid dissociation equilibrium (acidity) and used as, for example, an index for determining the presence of a proton ($H^+$) that is important in a chemical reaction in biomolecules.

A variety of techniques for predicting $pK_a$ have been therefore studied. Such techniques are broadly classified into two types: a technique based on the thermodynamic theory and a technique involving approximation by a function of a physical property that is a variable.

The former technique enables theoretical calculation, and the latter technique enables generally fast prediction.

In the technique based on the thermodynamic theory, however, not only the prediction is greatly affected by the number and position of water molecules located near a target molecule, but also highly accurate calculation is demanded to obtain good result (see Junming Ho, Michelle L. Coote, "A universal approach for continuum solvent pKa calculations: are we there yet?", Theor Chem Acc, pp.3-21, 2010). Fast prediction has been therefore still under development. Thus, such a technique is impractical for analysis of macromolecules and screening of mass data.

In the technique involving approximation by a function of a physical property that is a variable, an approach of using a variety of physical properties has been made to enable highly accurate prediction. In an example of such an approach, the electrical charges of a hydrogen atom (H) dissociated into a proton and oxygen atom (O) directly bonded to H and the distance therebetween are used as variables (see Jahanbakhsh Ghasemi, Saadi Saaidpour, Steven D. Brown, "QSPRstudy for estimation of acidity constants of some aromatic acids derivatives using multiple linear regression (MLR) analysis", Journal of Molecular Structure, THEOCHEM, pp. 27-32, 2007). In the case of using such variables, however, another function expression is entailed on the basis of, for instance, the type of the acid of a target molecule; in addition, all function expressions have not given highly accurate results (see Mario J. Citra, "ESTIMATING THE pKa OF PHENOLS, CARBOXYLIC ACIDS AND ALCOHOLS FROM SEMI-EMPIRICAL QUANTUM CHEMICAL METHODS", Chemosphere, Vol. 38, No. 1, pp. 191-206, 1999). Hence, such a technique is unsuitable for analysis of novel molecules. Soriano E. et al. (2004) "Computational determination of pKa values. A comparison of different theoretical approaches and a novel procedure",JOURNAL OF MOLECULAR STRUCTURE (THEOCHEM), vol. 684, no. 1-3, pages 121-12 teaches estimating pKa values of different series of imidazol-1-ylalcanoic acid derivatives using combinations of semiempirical or ab initio methods and two semiempirical solvation models SM2 and SM5.4.

Saracino G. A. A. et al. (2003) "Absolute pKa determination for carboxylic acids using density functional theory and the polarizable continuum model", CHEMICAL PHYSICS LETTERS, vol. 373, no. 3-4, pages 411-415 discloses the computation of absolute pKa values of a number of carboxylic acids using a Born-Haber cycle, experimental value for the proton solvation energy and completely ab initio structures, energies and harmonic frequencies of acids and conjugated basis both in vacuo and in aqueous solution.

SUMMARY

It is desirable to enable the predicted value of $pK_a$ to be applied to analysis of macromolecules, screening of mass data, and analysis of novel molecules.

Aspects and embodiments of the invention are set out in the appended claims.

The advantages of the invention will be realized and attained by means of the elements and combinations particularly pointed out in the claims.

It is to be understood that both the foregoing general description and the following detailed description are exemplary and explanatory and are not restrictive of the invention, as claimed.

BRIEF DESCRIPTION OF DRAWINGS

FIG. 1 illustrates an acid dissociation constant $pK_a$;
FIG. 2 illustrates an example of a method for predicting $pK_a$;
FIG. 3 illustrates another example of a method for predicting $pK_a$;
FIG. 4 illustrates a configuration example of a molecular design support system according to an embodiment;
FIG. 5 illustrates the hardware configuration of a computer apparatus;

FIG. 6 illustrates an example of the functional configuration of the molecular design support system;
FIG. 7 illustrates a pair type;
FIG. 8 illustrates an example of a process for predicting an acid dissociation constant;
FIG. 9 illustrates the molecular structure of formic acid;
FIG. 10A illustrates results of analysis by related art; and
FIG. 10B illustrates results of analysis by the embodiment.

DESCRIPTION OF EMBODIMENT

An embodiment will hereinafter be described with reference to the drawings. First of all, a typical method for predicting an acid dissociation constant $pK_a$ will now be described.

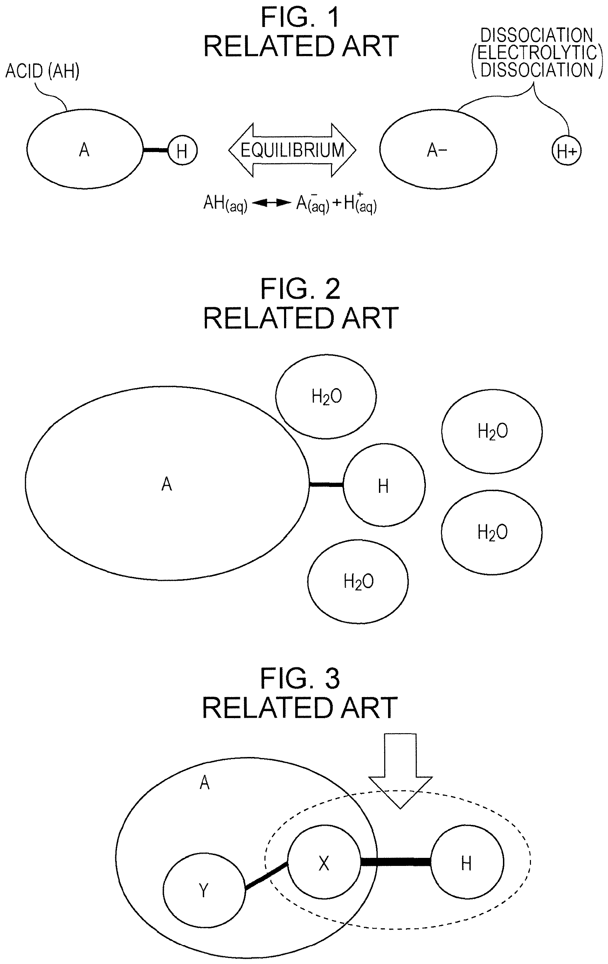

FIG. 1 illustrates an acid dissociation constant $pK_a$. $pK_a$ is a constant that represents acid dissociation equilibrium as illustrated in FIG. 1 and represented by Formula 1.

$$pK_a = -\log K_a \quad \text{where} \quad K_a = \frac{\left[A^-\right]\left[H^+\right]}{\left[AH\right]} \tag{1}$$

$pK_a$ serves as an index for determining the presence of a proton ($H^+$) that is important in a chemical reaction in biomolecules.
In a known prediction technique, $pK_a$ is obtained from a definition equation. FIG. 2 illustrates an example of a method for predicting $pK_a$. In the prediction technique by a definition equation, $pK_a$ is defined as a value that is in proportion to a change $\Delta G$ in free energy as represented by Formula 2.

$$AH_{(aq)} \overset{\Delta G}{\leftrightarrow} A^-_{(aq)} + H^+_{(aq)} \quad \Rightarrow \quad pK_a \propto \Delta G \tag{2}$$

In such a prediction technique, a prediction result depends on the number and position of water molecules located near an acid AH illustrated in FIG. 2, and highly accurate calculation is therefore demanded. Hence, such a technique is unsuitable for analysis of macromolecules and screening of mass data.
FIG. 3 illustrates another example of a method for predicting $pK_a$. In a prediction technique illustrated in FIG. 3, only the physical properties related to a hydrogen H and an atom X of a molecule A (hereinafter referred to as atom H-X) are utilized. In particular, $pK_a$ is predicted on the basis of an estimated formula (Formula 3) in which the physical properties related to the atom H-X ($d_1, d_2,..., d_N$) are used as variables.

$$pK_a \cong f(d_1, d_2, \cdots, d_N) \tag{3}$$

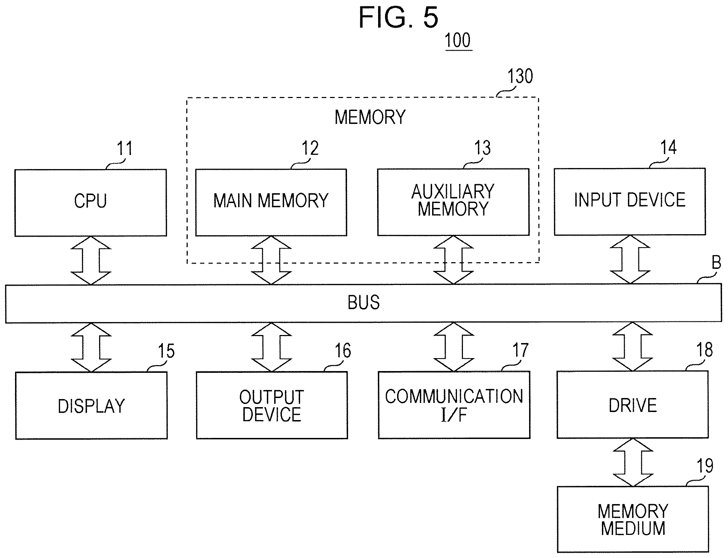

In this prediction method, however, an estimated formula greatly varies depending on the types of molecules; hence, such a prediction technique is impractical for analysis of novel molecules and prediction of $pK_a$ in a multistep oxidation reaction.
This embodiment provides a prediction apparatus and prediction method in which an index based on the electron density of an interatomic bond is used to enable fast and highly accurate prediction of the acid dissociation constant $pK_a$ of a molecule regardless of the types of molecules.

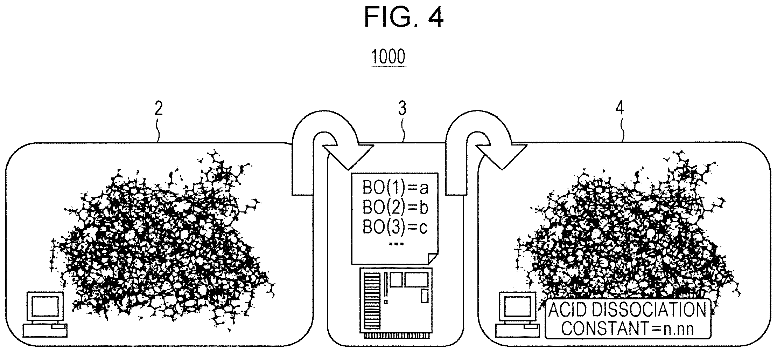

FIG. 4 illustrates a configuration example of a molecular design support system according to the embodiment. With reference to FIG. 4, a molecular design support system 1000 includes a molecular structure design unit 2, an acid dissociation constant prediction unit 3, and a prediction result display unit 4.
The combination of the molecular structure design unit 2, acid dissociation constant prediction unit 3, and prediction result display unit 4, namely, the molecular design support system 1000 may be in the form of one computer apparatus. Alternatively, the molecular structure design unit 2, the acid dissociation constant prediction unit 3, and the prediction result display unit 4 may be in the form of independent computer apparatuses. Furthermore, the combination of the acid dissociation constant prediction unit 3 and the prediction result display unit 4 may be in the form of one computer apparatus; such a computer apparatus corresponds to an acid dissociation constant prediction apparatus.
A computer apparatus that serves as the molecular design support system 1000 has, for example, a hardware configuration illustrated in FIG. 5. FIG. 5 illustrates the hardware configuration of a computer apparatus. With reference to FIG. 5, a computer apparatus 100 includes a central processing unit (CPU) 11, a main memory 12, an auxiliary memory 13, an input device 14, a display 15, an output device 16, a communication interface I/F 17, and a drive 18 and is connected to a bus B.

The CPU 11 controls the computer apparatus 100 on the basis of a program stored in the main memory 12. An example of the main memory 12 is a random access memory (RAM), and the main memory 12 stores, for instance, a program that is to be executed by the CPU 11, data used for processing by the CPU 11, and data obtained through processing by the CPU 11. Part of the storage area of the main memory 12 is allocated to a working area used for processing by the CPU 11.

A hard disk drive is used as the auxiliary memory 13 and stores data such as programs used for carrying out various processing. Some of the programs stored in the auxiliary memory 13 are loaded by the main memory 12 and executed by the CPU 11, thereby carrying out various processing. A memory 130 includes the main memory 12 and/or the auxiliary memory 13.

The input device 14 includes, for example, a mouse and a keyboard and is handled by users for inputting a variety of information used for processing by the computer apparatus 100. On the display 15, a variety of useful information is displayed under control of the CPU 11. The output device 16 includes, for instance, a printer and serves to output a variety of information in response to instructions from users. The communication I/F 17 is connected to, for example, internet or local area network (LAN) and serves to control communication with an external apparatus. The communication by the communication I/F 17 is not limited to wireless communication or wire communication.

A program used for processing by the computer apparatus 100 is provided for the computer apparatus 100 from a memory medium 19 such as a compact disc read-only memory (CD-ROM). In particular, once the memory medium 19 storing a program is placed on the drive 18, the drive 18 reads the program from the memory medium 19, and the read program is installed in the auxiliary memory 13 through the bus B. When the program installed in the auxiliary memory 13 is started, the CPU 11 starts processing on the basis of the program. The medium storing a program is not limited to CD-ROMs, and any computer-readable medium may be used. Examples of a computer-readable memory medium other than CD-ROMs include digital versatile discs (DVDs), portable memory media such as a USB memory, and semiconductor memories such as a flash memory.

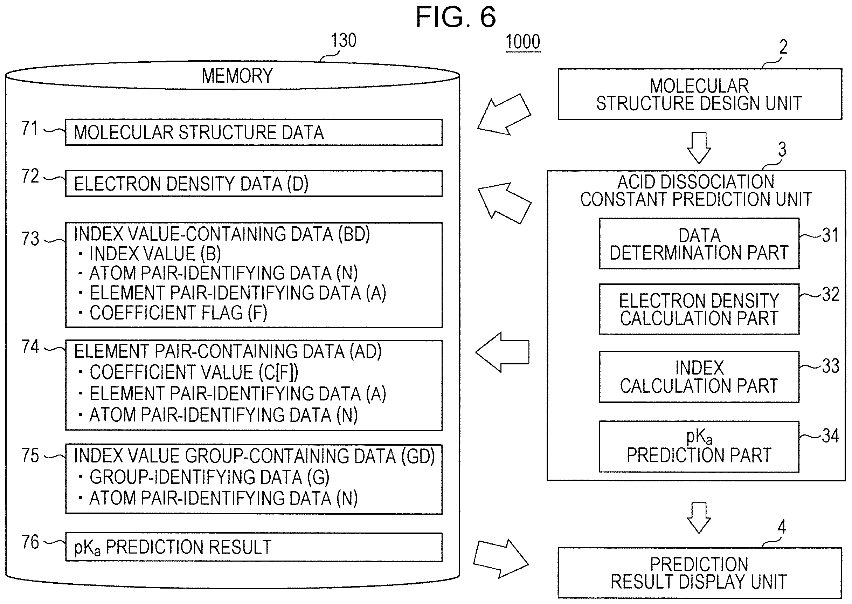

FIG. 6 illustrates an example of the functional configuration of a molecular design support system. The molecular design support system 1000 includes the molecular structure design unit 2, the acid dissociation constant prediction unit 3, and the prediction result display unit 4 as illustrated in FIG. 6. The memory 130 stores molecular structure data 71, electron density data (D) 72, index value-containing data (BD) 73, element pair-containing data (AD) 74, index value group-containing data (GD) 75, and a $pK_a$ prediction result 76.

The molecular structure design unit 2 helps users to design a molecular structure. The datum of the molecular structure designed by users (hereinafter referred to as molecular structure data 71) is stored in the memory 130. The molecular structure data 71 include the coordinates of atoms constituting a molecule and information of a dissociated proton.

The acid dissociation constant prediction unit 3 highly accurately predicts an acid dissociation constant $pK_a$ and includes a data determination part 31, an electron density calculation part 32, an index calculation part 33, and a $pK_a$ prediction part 34.

In the data determination part 31, atom pairs of the molecular structure designed by users are formed with reference to the molecular structure data 71 and stored in the element pair-containing data (AD) 74 of the memory 130.

The electron density calculation part 32 calculates the molecular orbital and calculates the electron density of the entire molecule. Electron density data (D) that represents the calculated electron density is stored in the memory 130.

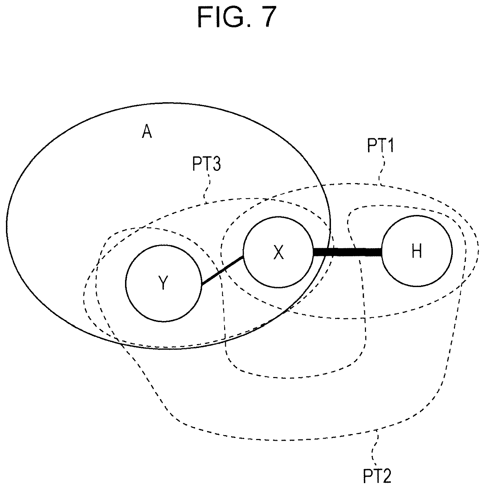

In an example of a simple molecular structure in FIG. 7, a target proton H and a molecule A are illustrated; in the molecule A, X is an atom directly bonded to the target proton H, and Y is an atom which is not bonded to the target proton H. The atom Y may be multiple. Atom pairs formed in the calculation of electron density are classified into the following pair types: a pair type PT1 "H-X", namely, an atom pair consisting of the target proton H and the atom X directly bonded to the target proton H; a pair type PT2 "H-Y" (all atoms other than the atom X contained in the molecule A), namely, an atom pair consisting of the target proton H and the atom Y other than the atom X directly bonded to the target proton H; a pair type PT 3 " X-Y", namely, an atom pair including the atom X directly bonded to the target proton H, not including the target proton H; and a pair type PT 4 that is an atom pair other than the pair types PT1 to PT 3.

On the basis of the electron density obtained by the electron density calculation part 32, the index calculation part 33 calculates an index value that represents bond strength.

Through the processing by the electron density calculation part 32 and the index calculation part 33, an index value (Formula 4) is determined from the electron density ($D_{ab}$) (Formula 5) between atoms a and b with reference to I. Mayer, "Bond Order and Valence Indices: A Personal Account", Journal of Computational Chemistry Special Issue, Vol. 28, No. 1, Wiley InterScience, Wiley Periodicals, Inc., pp. 204-221, 2007.

$$B_{\mathrm{ab}} = W_{ab} = \sum_{\mu \in a} \sum_{\nu \in b} \left| D_{\mu\nu} \right|^2 \qquad (4)$$

where

$$D_{\mu\nu} = 2 \sum_i^{occ.} C_{\mu i} C_{\nu i}^* \qquad (5)$$

In Formula 4, an atom pair is represented by atoms a and b.

The $pK_a$ prediction part 34 weights the index values of atom pairs related to the target proton H and atom X directly bonded to the target proton H in terms of the types of the element pairs. The $pK_a$ prediction part 34 determines a $pK_a$ estimated formula (Formula 6) on the basis of the index value group-containing data (GD) 75.

$$pK_a \cong \mathrm{f}\left(C_{\mathrm{XY}}B_{\mathrm{XY}}, C_{\mathrm{YH}}B_{\mathrm{YH}}, \cdots, C_{\mathrm{XH}}B_{\mathrm{XH}}\right) \qquad (6)$$

Weighed index values (B) are derived from a data set including the element pair-identifying data (A) and atom pair-identifying data (N) of atom pairs included in groups based on the index value group-containing data (GD) 75, thereby determining the $pK_a$ estimated formula (Formula 6).

The prediction result display unit 4 serves to display result of the prediction of $pK_a$ by the $pK_a$ prediction part 34 on the display 15.

The index value-containing data (BD) 73 has a data structure storing the index values of interatomic bonds, which have been determined on the basis of electron density, and includes an index value (B), atom pair-identifying data (N), element pair-identifying data (A), and a coefficient flag (F).

The element pair-containing data (AD) 74 has a data structure storing a coefficient value specific to two elements concerning bonding in intermolecular bonds and includes a coefficient value (C[F]), element pair-identifying data (A), and atom pair-identifying data (N). The coefficient value (C[F]) is determined for each element pair in advance.

The index value group-containing data (GD) 75 has a data structure storing the groups of atom pairs related to the target proton H and the atom X directly bonded to the target proton H and includes group-identifying data (G) and atom pair-identifying data (N).

The items related to weighting of index values based on such data structures are herein specified as follows:

Index value group-containing data GD of group number G: GD[G]
Atom pair-identifying data N of GD[G]: GD[G]->N
Index value-containing data BD to which the group number GD[G]->N belongs: BD[GD[G]->N]
Index value data B of BD[GD[G]->N]: BD[GD[G]->N]->B
Coefficient flag F of BD[GD[G]->N]: BD[GD[G]->N]->F
Element pair-identifying data belonging to BD[GD[G]->N]: BD[GD[G]->N]->A
Element pair-containing data AD to which BD[GD[G]->N]->A belongs: AD[BD[GD[G]->N]->A]
Coefficient value data C of AD[BD[GD[G]->N]->A]: AD[BD[GD[G]->N]->A]->C[BD[GD[G]->N]->F]
Index value data B of BD[GD[G]->N]: BD[GD[G]->N]->B
Hence, the weighting of an index value based on a coefficient value is obtained by the following formula: AD[BD[GD[G]->N]->A]->C[BD[GD[G]->N]->F] $\times$ BD[GD[G]->N]->B.

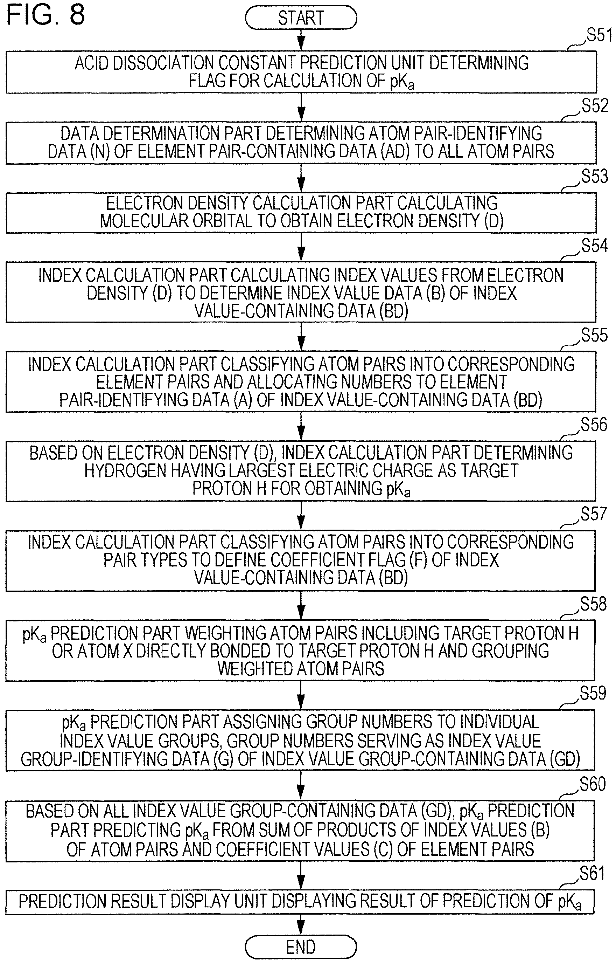

An example of a process for predicting an acid dissociation constant according to the embodiment will now be described with reference to FIG. 8. FIG. 8 illustrates an example of a process for predicting an acid dissociation constant. With reference to FIG. 8, in the acid dissociation constant prediction unit 3, a flag is determined for calculation of $pK_a$ and starts the process for predicting an acid dissociation constant (step S51). In the acid dissociation constant prediction unit 3, the data determination part 31 determines the atom pair-identifying data (N) of the element pair-containing data (AD) to all atom pairs (step S52). The electron density calculation part 32 calculates a molecular orbital to obtain electron density (D) (step S53).

Then, the index calculation part 33 calculates index values from the electron density (D) to determine the index value data (B) of the index value-containing data (BD) (step S54). The index calculation part 33 classifies the atom pairs into corresponding element pairs and allocates numbers to the element pair-identifying data (A) of the index value-containing data (BD) (step S55).

On the basis of the electron density (D), the index calculation part 33 determines the hydrogen having the largest electric charge as the target proton H for obtaining $pK_a$ (step S56). The index calculation part 33 classifies the atom pairs into corresponding pair types to define the coefficient flag (F) of the index value-containing data (BD) (step S57).

The $pK_a$ prediction part 34 weights atom pairs including the target proton H or the atom X directly bonded to the target proton H and then groups the weighted atom pairs (step S58). Atom pairs weighted into the same category are classified into the same group.

The $pK_a$ prediction part 34 assigns group numbers to the individual index value groups (step S59). The group numbers are determined as the index value group-identifying data (G) of the index value group-containing data (GD).

On the basis of all index value group-containing data (GD), the $pK_a$ prediction part 34 predicts $pK_a$ from the sum of products (Formula 6) of the index values (B) of the atom pairs and the coefficient values (C) of the element pairs (step S60).

Result of the prediction of $pK_a$ is displayed on the display 15 by the prediction result display unit 4 (step S61). In the displaying of the result of the prediction of $pK_a$ on the display 15, the image of the molecular structure including the target proton H may be displayed on the display 15.

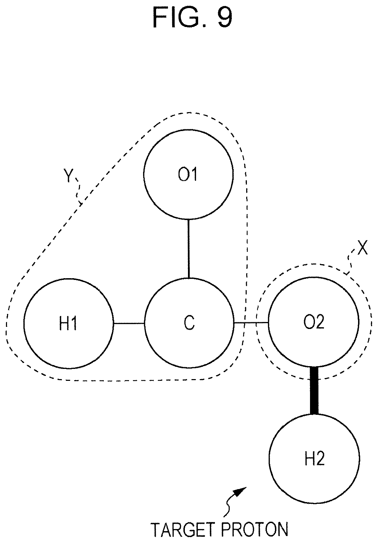

Specific examples of the above-mentioned process for predicting an acid dissociation constant will now be described, in which formic acid is employed as an example. FIG. 9 illustrates the molecular structure of formic acid. Circles represent atomic particles, and the identification names of atoms C, 01, 02, H1, and H2 are given inside the circles to uniquely identify the atoms in the molecular structure.

The atom "H2" is the target proton, the atom "O2" directly bonded to the target proton is an atom X, and the atoms "C", "O1", and "H1" not bonded to the target proton H are atoms Y.

The molecular structure data 71 that represents the molecular structure of formic acid is stored in the memory 130.

The data decision part 31 creates atom pairs from the molecular structure of formic acid on the basis of the molecular structure data 71. To each atom pair found in the molecular structure of formic acid, atom pair-identifying data N (N is an integer) is allocated. The atom pairs are identifiable in the index value-containing data BD[N]. Examples of the atom pairs found in the molecular structure of formic acid in FIG. 9 and allocation of the atom pair-identifying data N are as follows:

C-O1: 1
C-O2: 2
C-H1: 3
C-H2: 4
O1-O2: 5
O1-H1: 6
O1-H2: 7
O2-H1: 8
O2-H2: 9
H1-H2: 10

From the identified atom pairs, the data decision part 31 extracts element pairs; in this case, different atom pairs which consist of the same elements are regarded as the same element pair. To each element pair identified through the extraction, the element pair-identifying data A (A is an integer) is allocated. The element pairs are identifiable in the element pair-containing data AD [A]. Examples of the element pairs identified in the atom pairs in the molecular structure of formic acid in FIG. 9 and allocation of the element pair-identifying data A are as follows:

C-O: 1
C-H: 2
O-O: 3
O-H: 4
H-H: 5

In the element pairs and atom pairs which have been identified as described above, the element pairs and the atom pairs corresponding thereto are registered in the form of AD[A]->N; an example thereof in the molecular structure of formic acid in FIG. 9 is as follows:

AD[1]->N[1] = 1, AD[1]->N[2] = 2

AD[2]->N[1] = 3, AD[2]->N[2] = 4
AD[3]->N[1] = 5
AD[4]->N[1] = 6, AD[4]->N[2] = 7, AD[4]->N[3] = 8, AD[4]->N[4] = 9
AD[5]->N[1] = 10

Then, the electron density calculation part 32 calculates a molecular orbital and calculates the electron density of each atom pair by Formula 5. The index calculation part 33 obtains the index value B of each atom pair from the obtained electron density by Formula 4.

The index values B of the atom pairs are determined in the index value-containing data (BD) 73 as follows:

BD[1]->B = $B_{C-O1}$
BD[2]->B = $B_{C-O2}$
BD[3]->B = $B_{C-H1}$
BD[4]->B = $B_{C-H2}$
BD[5]->B = $B_{O1-O2}$
BD[6]->B = $B_{O1-H1}$
BD[7]->B = $B_{O1-H2}$
BD[8]->B = $B_{O2-H1}$
BD[9]->B = $B_{O2-H2}$
BD[10]->B = $B_{H1-H2}$

The electron density calculation part 32 allocates the element pair-identifying data (A) of the atom pairs to the index value-containing data (BD) 73.

The element pair-identifying data (A) of the atom pairs is allocated to the index value-containing data (BD) 73 as follows:

BD[1,2]->A = 1
BD[3,4]->A = 2
BD[5]->A = 3
BD[6,7,8,9]->A = 4
BD[10]->A = 5

The electron density calculation part 32 determines the pair type of each atom pair.

In particular, the coefficient flags (F) of the index value-containing data (BD) 73 are determined as follows:

BD[1]->F = 4
BD[2]->F = 3
BD[3]->F = 4
BD[4]->F = 2
BD[5]->F = 3
BD[6]->F = 4
BD[7]->F = 2
BD[8]->F = 3
BD[9]->F = 1
BD[10]->F = 2

In order to consider the electron density of the entire molecule, the $pK_a$ prediction part 34 groups atom pairs including the atom "H2" defined as the target proton (target proton itself) or the atom "O2" (atom directly connected to target proton) in terms of weighting. With reference to the coefficient flags (F) that represent the pair types, the categories of the weighting are determined. The atom pairs of the same pair type are weighted into the same category. In this case, the atom pairs of the coefficient flag (F) "4" are not considered.

The atom pairs are classified into the categories of weighting as follows:

Weighting 1: O2-O1, O2-H1, and O2-C
Weighting 2: H2-O1, H2-H1, and H2-C
Weighting 3: O2-H2

The $pK_a$ prediction part 34 classifies the atom pairs grouped in terms of weighting into index value groups. In the index value groups, the target proton or the atom X directly bonded to the target proton is paired with the element corresponding to the atom combined therewith (hereinafter referred to as index value pair), thereby grouping the weighted atom pairs. The weighted atom pairs correspond to the atom pairs having the coefficient flags (F) "1", "2", and "3".

Identification data is allocated to each index value group, for example, as follows:

O2-O: 1
O2-H: 2
O2-C: 3
H2-O: 4
H2-H: 5
H2-C: 6

These numbers are determined as the group-identifying data (G) of the index value group-containing data (GD) 75.

Then, the $pK_a$ prediction part 34 multiplies the index value (B) of the atom pair (O2-X, H2-X, or O2-H2) by the coefficient value (C[F]) of the element pair in each index value group GD [G]. The coefficient (C[F]) of the element pair is specified by the number "1", "2", or "3" of the coefficient flag (F).

The index value group "1" has one atom pair. The atom pair "O1-O2" has atom pair-identifying data "5", and the element pair "O-O" thereof has element pair-identifying data "3". In addition, the coefficient flag that specifies the weighting of the atom pair "O1-O2" is "3".

In particular, the following relationship is obtained:

GD[1]->N[1] = 5, BD[5]->A = 3, BD[5]->F = 3
therefore,
AD[3]->C[3] $\times$ BD[5]->B
accordingly,
$C_{O-O}(3) \times B_{O1-O2}$.

Similarly, the index value group "2" has two atom pairs. The following relationships are obtained:

GD[2]->N[1] = 8, BD[8]->A = 4, BD[8]->F = 3
therefore,
AD[4]->C[3] $\times$ BD[8]->B
accordingly,
$C_{O-H}(3) \times B_{O2-H1}$; and
GD[2]->N[2] = 9, BD[9]->A = 4, BD[9]->F = 1
therefore
AD[4]->C[1] $\times$ BD[9]->B
accordingly
$C_{O-H}(1) \times B_{O2-H2}$.

The index value group "3" has one atom pair. In particular, the following relationship is obtained:

GD[3]->N[1] = 2, BD[2]->A = 1, BD[2]->F = 3
therefore,
AD[1]->C[3] $\times$ BD[2]->B
accordingly,
$C_{C-O}(1) \times B_{C-O2}$.

The index value group "4" has two atom pairs. In particular, the following relationships are obtained:

GD[4]->N[1] = 7, BD[7]->A = 4, BD[7]->F = 2
therefore,
AD[4]->C[2] $\times$ BD[7]->B
accordingly,
$C_{C-H}(2) \times B_{O1-H2}$; and
GD[4]->N[2] = 9, BD[9]->A = 4, BD[9]->F = 1
therefore,
AD[4]->C[1] $\times$ BD[9]->B

accordingly,
$C_{O\text{-}H}(1) \times B_{O2\text{-}H2}$.

The index value group "5" has one atom pair. In particular, the following relationship is obtained:

GD[5]->N[1] = 10, BD[10]->A = 5, BD[10]->F = 2
therefore,
AD[5]->C[2] $\times$ BD[10]->B
accordingly,
$C_{H\text{-}H}(2) \times B_{H1\text{-}H2}$.

The index value group "6" has one atom pair. In particular, the following relationship is obtained:

GD[6]->N[1] = 4, BD[4]->A = 2, BD[4]->F = 2
therefore,
AD[2]->C[2] $\times$ BD[4]->B
accordingly,
$C_{C\text{-}H}(2) \times B_{C\text{-}H2}$.

The above-mentioned results of the multiplication are added together.

$$
\begin{aligned}
pK_a = {} & constant + C_{O\text{-}H}(1) \times B_{O2\text{-}H2} \\
& + C_{O\text{-}O}(3) \times B_{O1\text{-}O2} \\
& + C_{O\text{-}H}(3) \times B_{O2\text{-}H1} \\
& + C_{C\text{-}O}(3) \times B_{C\text{-}O2} \\
& + C_{O\text{-}H}(2) \times B_{O1\text{-}H2} \\
& + C_{H\text{-}H}(2) \times B_{H1\text{-}H2} \\
& + C_{C\text{-}H}(2) \times B_{C\text{-}H2}
\end{aligned}
$$

This process enables prediction of an acid dissociation constant $pK_a$ based on 02-H2 of formic acid.

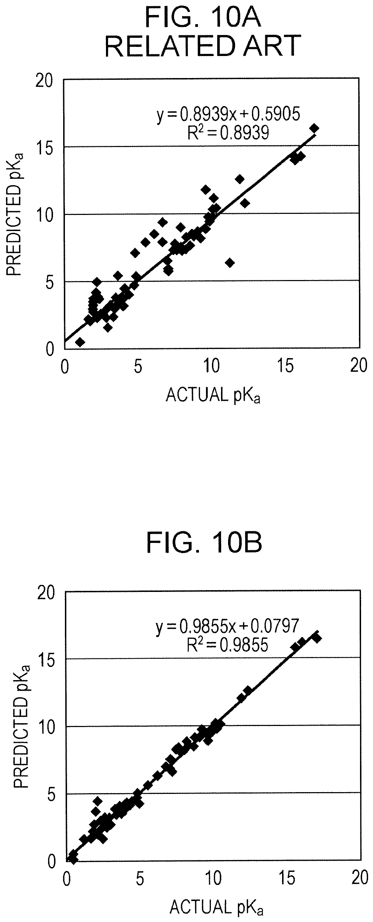

A process for predicting an acid dissociation constant according to the embodiment has been described with reference to an example of the molecular structure of formic acid in FIG. 9; furthermore, the inventor has obtained correlations between actual values and predicted values of 103 molecules that are analysis subjects. FIGs. 10A and 10B illustrate effects of the embodiment. FIG. 10A illustrates results of analysis by the technique disclosed in Jahanbakhsh Ghasemi, Saadi Saaidpour, Steven D. Brown, "QSPRstudy for estimation of acidity constants of some aromatic acids derivatives using multiple linear regression (MLR) analysis", Journal of Molecular Structure, THEOCHEM, pp. 27-32, 2007. In the analysis in FIG. 10A, the relationship of x = 121.4191 - 240.451 pchgH - 43.4984 bl(O-H) + 24.30716 pchgO is provided.

FIG. 10B illustrates results of analysis by the embodiment. In FIG. 10B, electron density is defined by Austin model 1 (AM1) disclosed in Jahanbakhsh Ghasemi, Saadi Saaidpour, Steven D. Brown, "QSPRstudy for estimation of acidity constants of some aromatic acids derivatives using multiple linear regression (MLR) analysis", Journal of Molecular Structure, THEOCHEM, pp. 27-32, 2007; and index values are calculated on the basis of Index of Wiberg (Formula described above) disclosed in I. Mayer, "Bond Order and Valence Indices: A Personal Account", Journal of Computational Chemistry Special Issue, Vol. 28, No. 1, Wiley InterScience, Wiley Periodicals, Inc., pp. 204-221, 2007.

A correlation $R^2$ is approximately 0.89 in the related art as illustrated in FIG. 10A; in contrast, a correlation $R^2$ is approximately 0.99 in the embodiment as illustratec in FIG. 10B, which elucidates that the embodiment may provide highly accurate results even through a calculation that is as fast as the calculation by the technique disclosed in Jahanbakhsh Ghasemi, Saadi Saaidpour, Steven D. Brown, "QSPRstudy for prediction of acidity constants of some aromatic acids derivatives using multiple linear regression (MLR) analysis", Journal of Molecular Structure, THEOCHEM, pp. 27-32, 2007.

As described above, an index (for example, bond order) based on the electron density of an intermolecular bond is utilized, which enables fast and highly accurate prediction of the acid dissociation constant pKa of a target molecule regardless of the types of molecules.

**Claims**

1. An apparatus for predicting an acid dissociation constant of a molecule, the apparatus comprising:

    a molecular structure design unit (2) configured to design the molecule;
    an acid dissociation constant prediction unit (3) configured to:

        calculate molecular orbital of the designed molecule to obtain electron density (D) of the designed molecule,
        calculate index value-containing data (73) including index values (B) indicating a bond strength of respective atom pairs of respective interatomic bonds of the designed molecule based on the electron density (D),
        determine a hydrogen having the largest electric charge in the designed molecule as a target proton (H) based on the electron density (D),
        set a coefficient value (C[F]) included in element pair-containing data (74) to each of the atom pairs according to a pair type of each of the atom pairs, wherein the pair type of the atom pairs includes a first type of an atom pair between the target proton (H) and a first atom directly bonded to the target proton (H), a second type of an atom pair between the target proton (H) and a second atom not directly bonded to the target proton (H) and a third type of an atom pair between the first atom and the second atom,
        classify the atom pairs into corresponding pair types, and
        predict an acid dissociation constant (pKa) of the designed molecule by calculating a sum of products of the index values of the atom pairs and the coefficient values (C[F]) of the element pairs for each of the pair types;

    a memory (130) configured to store molecular structure data (71) defining the designed molecule, electron density data (72) defining the electron density (D), the index value-containing data (73), the element pair-containing data (74), index value group-containing data (75) including data defining the corresponding pair types, and the predicted acid dissociation constant (pKa); and
    a prediction result display unit (4) configured to display the predicted acid dissociation constant (pKa) using a display device.

2. The apparatus according to claim 1, wherein the prediction result display unit (4) is configured to display a structure of the designed molecule including the target proton (H) with the predicted acid dissociation constant (pKa) using the display device.

3. The apparatus according to claim 2, wherein each of the index values of the respective atoms pairs is weighted by the respective coefficient values (C[F]).

4. The apparatus according to claim 2, wherein the proton corresponds to a hydrogen atom dissociated in the designed molecule.

5. The apparatus according to claim 2, wherein the predicting is performed by a function in which the coefficient value (C[F]) included in the element pair-containing data (74) and that index value (B) included in the index value-containing data (73) are used.

6. A computer-implemented method for predicting an acid dissociation constant, the method comprising:

    designing a molecule;
    calculating, by a computer, electron density (D) of the designed molecule;
    calculating index value-containing data (73) including index values (B) indicating a bond strength of respective atom pairs of respective interatomic bonds of the designed molecule based on the electron density (D);
    determining a hydrogen having the largest electric charge in the designed molecule as a target proton (H) based on the electron density (D);
    setting a coefficient value (C[F]) included in element pair-containing data (74) to each of the atom pairs according to a pair type of each of the atom pairs, wherein the pair type of the atom pairs includes a first type of an atom pair between the target proton (H) and a first atom directly bonded to the target proton (H), a second type of an atom pair between the target proton (H) and a second atom not directly bonded to the target proton (H) and a third type of an atom pair between the first atom and the second atom;

classifying the atom pairs into corresponding pair types; predicting an acid dissociation constant (pKa) of the designed molecule by calculating a sum of products of the index values and the coefficient values (C[F]) of the atom pairs for each of the pair types;

storing, in a memory (130), molecular structure data (71) defining the designed molecule, electron density data (72) defining the electron density (D), the index value-containing data (73), the element pair-containing data (74), index value group-containing data (75) including data defining the corresponding pair types, and the predicted acid dissociation constant (pKa); and

displaying the predicted acid dissociation constant (pKa) using a display device.

7. A computer-readable storage medium that stores a program which, when executed by a computer, causes the computer to carry out a method for predicting an acid dissociation constant according to claim 6.

**Patentansprüche**

1. Vorrichtung zur Vorhersage einer Säuredissoziationskonstante eines Moleküls, wobei die Vorrichtung umfasst:

eine Molekularstruktur-Designeinheit (2), die dazu ausgelegt ist, das Molekül zu entwerfen;
eine Säuredissoziationskonstanten-Vorhersageeinheit (3), die ausgelegt ist zum:

Berechnen des Molekülorbitals des entworfenen Moleküls, um die Elektronendichte (D) des entworfenen Moleküls zu erhalten,
Berechnen von Indexwert-enthaltenden Daten (73), die Indexwerte (B) aufweisen, die eine Bindungsstärke jeweiliger Atompaare jeweiliger interatomarer Bindungen des entworfenen Moleküls basierend auf der Elektronendichte (D) angeben,
Bestimmen eines Wasserstoffs mit der größten elektrischen Ladung in dem entworfenen Molekül als Zielproton (H) basierend auf der Elektronendichte (D),
Setzen eines Koeffizientenwerts (C[F]), der in Elementpaar-enthaltenden Daten (74) enthalten ist, für jedes der Atompaare gemäß einem Paartyp jedes der Atompaare, wobei der Paartyp der Atompaare einen ersten Typ eines Atompaares zwischen dem Zielproton (H) und einem ersten Atom, das direkt an das Zielproton (H) gebunden ist,
einen zweiten Typ eines Atompaares zwischen dem Zielproton (H) und einem zweiten Atom, das nicht direkt an das Zielproton (H) gebunden ist, und einen dritten Typ eines Atompaares zwischen dem ersten Atom und dem zweiten Atom aufweist, Klassifizieren der Atompaare in entsprechende Paartypen, und Vorhersagen einer Säuredissoziationskonstante (pKa) des entworfenen Moleküls durch Berechnen einer Summe von Produkten der Indexwerte der Atompaare und der Koeffizientenwerte (C[F]) der Elementpaare für jeden der Paartypen;

einen Speicher (130), der dazu ausgelegt ist, Molekularstrukturdaten (71), die das entworfene Molekül definieren, Elektronendichtedaten (72), die die Elektronendichte (D) definieren, die Indexwert-enthaltenden Daten (73), die Elementpaar-enthaltenden Daten (74), Indexwertgruppen-enthaltende Daten (75), die Daten aufweisen, die die entsprechenden Paartypen definieren, und die vorhergesagte Säuredissoziationskonstante (pKa) zu speichern; und
eine Vorhersageergebnis-Anzeigeeinheit (4), die dazu ausgelegt ist, die vorhergesagte Säuredissoziationskonstante (pKa) unter Verwendung einer Anzeigevorrichtung anzuzeigen.

2. Vorrichtung nach Anspruch 1, wobei die Vorhersageergebnis-Anzeigeeinheit (4) dazu ausgelegt ist, eine Struktur des entworfenen Moleküls, das das Zielprotons (H) mit der vorhergesagten Säuredissoziationskonstante (pKa) aufweist, unter Verwendung der Anzeigevorrichtung anzuzeigen.

3. Vorrichtung nach Anspruch 2, wobei jeder der Indexwerte der jeweiligen Atompaare mit den jeweiligen Koeffizientenwerten (C[F]) gewichtet wird.

4. Vorrichtung nach Anspruch 2, wobei das Proton einem in dem entworfenen Molekül dissoziierten Wasserstoffatom entspricht.

**5.** Vorrichtung nach Anspruch 2, wobei die Vorhersage durch eine Funktion durchgeführt wird, in der der Koeffizientenwert (C[F]), der in den Elementpaar-enthaltenden Daten (74) enthalten ist, und der Indexwert (B), der in dem Indexwert-enthaltenden Daten (73) enthalten ist, verwendet werden.

**6.** Computerimplementiertes Verfahren zur Vorhersage einer Säuredissoziationskonstante, wobei das Verfahren umfasst:

Entwerfen eines Moleküls;
Berechnen der Elektronendichte (D) des entworfenen Moleküls durch einen Computer;
Berechnen von Indexwert-enthaltenden Daten (73), die Indexwerte (B) aufweisen, die eine Bindungsstärke jeweiliger Atompaare jeweiliger interatomarer Bindungen des entworfenen Moleküls basierend auf der Elektronendichte (D) angeben;
Bestimmen eines Wasserstoffs mit der größten elektrischen Ladung in dem entworfenen Molekül als ein Zielproton (H) basierend auf der Elektronendichte (D);
Setzen eines Koeffizientenwerts (C[F]), der in Elementpaar-enthaltenden Daten (74) enthalten ist, für jedes der Atompaare gemäß einem Paartyp jedes der Atompaare, wobei der Paartyp der Atompaare einen ersten Typ eines Atompaares zwischen dem Zielproton (H) und einem ersten Atom, das direkt an das Zielproton (H) gebunden ist, einen zweiten Typ eines Atompaares zwischen dem Zielproton (H) und einem zweiten Atom, das nicht direkt an das Zielproton gebunden ist (H), und einen dritten Typ eines Atompaares zwischen dem ersten Atom und dem zweiten Atom aufweist;
Klassifizieren der Atompaare in entsprechende Paartypen;
Vorhersagen einer Säuredissoziationskonstante (pKa) des entworfenen Moleküls durch Berechnen einer Summe von Produkten der Indexwerte und der Koeffizientenwerte (C[F]) der Atompaare für jeden der Paartypen;
Speichern, in einem Speicher (130), von Molekülstrukturdaten (71), die das entworfene Molekül definieren, Elektronendichtedaten (72), die die Elektronendichte (D) definieren, die Indexwert-enthaltenden Daten (73), die Elementpaar-enthaltenden Daten (74), Indexwertgruppen-enthaltende Daten (75), die Daten aufweisen, die die entsprechenden Paartypen definieren, und die vorhergesagte Säuredissoziationskonstante (pKa); und
Anzeigen der vorhergesagten Säuredissoziationskonstante (pKa) unter Verwendung einer Anzeigevorrichtung.

**7.** Computerlesbares Speichermedium, das ein Programm speichert, das, wenn es von einem Computer ausgeführt wird, den Computer veranlasst, ein Verfahren zur Vorhersage einer Säuredissoziationskonstante nach Anspruch 6 auszuführen.

**Revendications**

**1.** Appareil pour prédire une constante de dissociation acide d'une molécule, l'appareil comprenant :

une unité de conception de structure moléculaire (2) configurée pour concevoir la molécule ;
une unité de prédiction de constante de dissociation acide (3) configurée pour :

calculer une orbitale moléculaire de la molécule conçue pour obtenir une densité électronique (D) de la molécule conçue,
calculer des données contenant des valeurs d'indice (73) comprenant des valeurs d'indice (B) indiquant une force de liaison de paires d'atomes respectives de liaisons interatomiques respectives de la molécule conçue sur la base de la densité électronique (D),
déterminer un hydrogène ayant la plus grande charge électrique dans la molécule conçue en tant que proton cible (H) sur la base de la densité électronique (D),
établir une valeur de coefficient (C[F]) incluse dans des données contenant une paire d'éléments (74) à chacune des paires d'atomes selon un type de paire de chacune des paires d'atomes, dans lequel le type de paire des paires d'atomes comprend un premier type d'une paire d'atomes entre le proton cible (H) et un premier atome directement lié au proton cible (H), un deuxième type d'une paire d'atomes entre le proton cible (H) et un second atome non directement lié au proton cible (H) et un troisième type d'une paire d'atomes entre le premier atome et le second atome,
classer les paires d'atomes en types de paire correspondants, et
prédire une constante de dissociation acide (pKa) de la molécule conçue en calculant une somme de produits des valeurs d'indice des paires d'atomes et des valeurs de coefficient (C[F]) des paires d'éléments pour chacun des types de paire ;

une mémoire (130) configurée pour stocker des données de structure moléculaire (71) définissant la molécule conçue, des données de densité électronique (72) définissant la densité électronique (D), les données contenant des valeurs d'indice (73), les données contenant une paire d'éléments (74), des données contenant un groupe de valeurs d'indice (75) comprenant des données définissant les types de paire correspondants, et la constante de dissociation acide prédite (pKa) ; et

une unité d'affichage de résultat de prédiction (4) configurée pour afficher la constante de dissociation acide prédite (pKa) en utilisant un dispositif d'affichage.

2. Appareil selon la revendication 1, dans lequel l'unité d'affichage de résultat de prédiction (4) est configurée pour afficher une structure de la molécule conçue comprenant le proton cible (H) avec la constante de dissociation acide prédite (pKa) en utilisant le dispositif d'affichage.

3. Appareil selon la revendication 2, dans lequel chacune des valeurs d'indice des paires d'atomes respectives est pondérée par les valeurs de coefficient respectives (C[F]) .

4. Appareil selon la revendication 2, dans lequel le proton correspond à un atome d'hydrogène dissocié dans la molécule conçue.

5. Appareil selon la revendication 2, dans lequel la prédiction est effectuée par une fonction dans laquelle la valeur de coefficient (C[F]) incluse dans les données contenant une paire d'éléments (74) et la valeur d'indice (B) incluse dans les données contenant des valeurs d'indice (73) sont utilisées.

6. Procédé mis en œuvre par ordinateur pour prédire une constante de dissociation acide, le procédé comprenant les étapes consistant à :

concevoir une molécule ;
calculer, par un ordinateur, une densité électronique (D) de la molécule conçue ;
calculer des données contenant des valeurs d'indice (73) comprenant des valeurs d'indice (B) indiquant une force de liaison de paires d'atomes respectives de liaisons interatomiques respectives de la molécule conçue sur la base de la densité électronique (D) ;
déterminer un hydrogène ayant la plus grande charge électrique dans la molécule conçue en tant que proton cible (H) sur la base de la densité électronique (D) ;
établir une valeur de coefficient (C[F]) incluse dans des données contenant une paire d'éléments (74) à chacune des paires d'atomes selon un type de paire de chacune des paires d'atomes, dans lequel le type de paire des paires d'atomes comprend un premier type d'une paire d'atomes entre le proton cible (H) et un premier atome directement lié au proton cible (H), un deuxième type d'une paire d'atomes entre le proton cible (H) et un second atome non directement lié au proton cible (H) et un troisième type d'une paire d'atomes entre le premier atome et le second atome ;
classer les paires d'atomes en types de paire correspondants ;
prédire une constante de dissociation acide (pKa) de la molécule conçue en calculant une somme de produits des valeurs d'indice et des valeurs de coefficient (C[F]) des paires d'atomes pour chacun des types de paire ;
stocker, dans une mémoire (130), des données de structure moléculaire (71) définissant la molécule conçue, des données de densité électronique (72) définissant la densité électronique (D), les données contenant des valeurs d'indice (73), les données contenant une paire d'éléments (74), des données contenant un groupe de valeurs d'indice (75) comprenant des données définissant les types de paire correspondants, et la constante de dissociation acide prédite (pKa) ; et
afficher la constante de dissociation acide prédite (pKa) en utilisant un dispositif d'affichage.

7. Support de stockage lisible par ordinateur qui stocke un programme qui, lorsqu'il est exécuté par un ordinateur, amène l'ordinateur à effectuer un procédé pour prédire une constante de dissociation acide selon la revendication 6.

# FIG. 1
# RELATED ART

ACID (AH)

DISSOCIATION
(ELECTROLYTIC DISSOCIATION)

A — H ⟨EQUILIBRIUM⟩ A− H+

$$AH_{(aq)} \longleftrightarrow A^-_{(aq)} + H^+_{(aq)}$$

# FIG. 2
# RELATED ART

$H_2O$

$H_2O$

A H

$H_2O$

$H_2O$

# FIG. 3
# RELATED ART

A

Y X H

EP 2 767 917 B1

# FIG. 4

1000

BO(1)=a
BO(2)=b
BO(3)=c
...

ACID DISSOCIATION
CONSTANT=n.nn

# FIG. 5

<u>100</u>

MEMORY 130

CPU 11

MAIN MEMORY 12

AUXILIARY MEMORY 13

INPUT DEVICE 14

BUS B

DISPLAY 15

OUTPUT DEVICE 16

COMMUNICATION I/F 17

DRIVE 18

MEMORY MEDIUM 19

EP 2 767 917 B1

# FIG. 6

# FIG. 7

# FIG. 8

START

S51

ACID DISSOCIATION CONSTANT PREDICTION UNIT DETERMINING
FLAG FOR CALCULATION OF $pK_a$

S52

DATA DETERMINATION PART DETERMINING ATOM PAIR-IDENTIFYING
DATA (N) OF ELEMENT PAIR-CONTAINING DATA (AD) TO ALL ATOM PAIRS

S53

ELECTRON DENSITY CALCULATION PART CALCULATING
MOLECULAR ORBITAL TO OBTAIN ELECTRON DENSITY (D)

S54

INDEX CALCULATION PART CALCULATING INDEX VALUES FROM ELECTRON
DENSITY (D) TO DETERMINE INDEX VALUE DATA (B) OF INDEX
VALUE-CONTAINING DATA (BD)

S55

INDEX CALCULATION PART CLASSIFYING ATOM PAIRS INTO CORRESPONDING
ELEMENT PAIRS AND ALLOCATING NUMBERS TO ELEMENT
PAIR-IDENTIFYING DATA (A) OF INDEX VALUE-CONTAINING DATA (BD)

S56

BASED ON ELECTRON DENSITY (D), INDEX CALCULATION PART DETERMINING
HYDROGEN HAVING LARGEST ELECTRIC CHARGE AS TARGET
PROTON H FOR OBTAINING $pK_a$

S57

INDEX CALCULATION PART CLASSIFYING ATOM PAIRS INTO CORRESPONDING
PAIR TYPES TO DEFINE COEFFICIENT FLAG (F) OF INDEX
VALUE-CONTAINING DATA (BD)

S58

$pK_a$ PREDICTION PART WEIGHTING ATOM PAIRS INCLUDING TARGET PROTON H
OR ATOM X DIRECTLY BONDED TO TARGET PROTON H AND GROUPING
WEIGHTED ATOM PAIRS

S59

$pK_a$ PREDICTION PART ASSIGNING GROUP NUMBERS TO INDIVIDUAL
INDEX VALUE GROUPS, GROUP NUMBERS SERVING AS INDEX VALUE
GROUP-IDENTIFYING DATA (G) OF INDEX VALUE GROUP-CONTAINING DATA (GD)

S60

BASED ON ALL INDEX VALUE GROUP-CONTAINING DATA (GD), $pK_a$ PREDICTION
PART PREDICTING $pK_a$ FROM SUM OF PRODUCTS OF INDEX VALUES (B)
OF ATOM PAIRS AND COEFFICIENT VALUES (C) OF ELEMENT PAIRS

S61

PREDICTION RESULT DISPLAY UNIT DISPLAYING RESULT OF PREDICTION OF $pK_a$

END

# FIG. 9

TARGET PROTON

## FIG. 10A
## RELATED ART

$y = 0.8939x + 0.5905$
$R^2 = 0.8939$

## FIG. 10B

$y = 0.9855x + 0.0797$
$R^2 = 0.9855$

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **JUNMING HO ; MICHELLE L. COOTE.** A universal approach for continuum solvent pKa calculations: are we there yet?. *Theor Chem Acc,* 2010, 3-21
- **JAHANBAKHSH GHASEMI ; SAADI SAAIDPOUR ; STEVEN D. BROWN.** QSPRstudy for estimation of acidity constants of some aromatic acids derivatives using multiple linear regression (MLR) analysis. *Journal of Molecular Structure, THEOCHEM,* 2007, 27-32
- **MARIO J. CITRA.** ESTIMATING THE pKa OF PHENOLS, CARBOXYLIC ACIDS AND ALCOHOLS FROM SEMI-EMPIRICAL QUANTUM CHEMICAL METHODS. *Chemosphere,* 1999, vol. 38 (1), 191-206
- **SORIANO E. et al.** Computational determination of pKa values. A comparison of different theoretical approaches and a novel procedure. *JOURNAL OF MOLECULAR STRUCTURE (THEOCHEM),* 2004, vol. 684 (1-3), 121-12

- **SARACINO G. A. A. et al.** Absolute pKa determination for carboxylic acids using density functional theory and the polarizable continuum model. *CHEMICAL PHYSICS LETTERS,* 2003, vol. 373 (3-4), 411-415
- Bond Order and Valence Indices: A Personal Account. **I. MAYER.** Journal of Computational Chemistry Special Issue. Wiley InterScience, Wiley Periodicals, Inc, 2007, vol. 28, 204-221
- QSPRstudy for estimation of acidity constants of some aromatic acids derivatives using multiple linear regression (MLR) analysis. **JAHANBAKHSH GHASEMI ; SAADI SAAIDPOUR ; STEVEN D. BROWN.** Journal of Molecular Structure. THEOCHEM, 2007, 27-32
- QSPRstudy for prediction of acidity constants of some aromatic acids derivatives using multiple linear regression (MLR) analysis. **JAHANBAKHSH GHASEMI ; SAADI SAAIDPOUR ; STEVEN D. BROWN.** Journal of Molecular Structure. THEOCHEM, 2007, 27-32